# FASCICULE DE BREVET EUROPEEN

(11) **EP 0 832 647 B1**
(45) Date de publication et mention de la délivrance du brevet: **04.06.2003**
(21) Numéro de dépôt: 97402142.0
(22) Date de dépôt: 16.09.1997
(51) Int. Cl.: A61K 9/107, A61K 7/00

(54) **Nouvelles compositions topiques sous forme d'émulsion fluide H/E à forte teneur en glycol pro-pénétrant**
Flüssige Öl-in-Wasser Emulsion mit einem erhöhten Gehalt an Glykolen zur topischen Anwendung
Topical oil-in-water fluid emulsions having a high content of glycol

(30) Priorité: 20.09.1996 FR 9611510
(43) Date de publication de la demande: 01.04.1998
(73) Titulaire: CENTRE INTERNATIONAL DE RECHERCHES DERMATOLOGIQUES GALDERMA (C.I.R.D. GALDERMA), 06560 Valbonne (FR)
(72) Inventeur: Preuilh, Isabelle, 06110 Le Canet (FR); Willcox, Nathalie, 06650 Le Rouret (FR)
(74) Mandataire: Tezier Herman, Béatrice

(56) Documents cités:
- EP-A- 0 268 164
- EP-A- 0 279 641
- EP-A- 0 347 225
- WO-A-94/17830
- DE-A- 2 646 435

## Description

La présente invention concerne une nouvelle composition sous forme d'émulsion fluide de type huile dans eau (H/E) pour une application topique, comprenant une forte teneur d'au moins un glycol pro-pénétrant, un système émulsionnant approprié et un agent actif.

Il existe actuellement de nombreuses compositions topiques comprenant un agent actif et une forte teneur en glycol, ce dernier favorisant la pénétration de l'agent actif dans la peau. Compte-tenu de la forte teneur en glycol pro-pénétrant, ces compositions sont formulées sous formes d'émulsions à forte teneur en phase grasse que l'on appelle aussi communément "lipocrèmes", sous formes de compositions anhydres que l'on appelle "onguents", sous forme de compositions fluides à forte teneur en solvants volatiles, tels que l'éthanol ou l'isopropanol, destinées à une applications sur le cuir chevelu, appellées également "lotions capillaires", ou encore sous forme d'émulsions H/E visqueuses, que l'on appelle aussi "crèmes H/E",

On connait par exemple des crèmes H/E comprenant un corticoïde et un fort pourcentage de propylène glycol (47,5 %) commercialisées sous la marque TEMOVATE® par la société GLAXO. Or, la stabilisation d'une formulation comprenant un tel pourcentage de glycol rend nécessaire l'emploi dans l'émulsion d'agents émulsifiants et stabilisants de type glycéryl stéarate ou PEG 100 stéarate ou encore d'agents stabilisants ou facteurs de consistance de type cire blanche ou alcool cétostéarylique qui conduisent à la formation d'une crème visqueuse, c'est à dire dont la viscosité est supérieure à 10 Pa.s (10000 centipoises, mesurée avec un appareil Brookfield modèle LVDV II + mobile n° 4, à une vitesse de 30 tours/mn pendant 30 secondes et à une température de 25 °C +/- 3 °C).

Pour faciliter l'application de compositions topiques comprenant un fort pourcentage de glycol, il était intéressant de disposer d'une nouvelle formulation stable de type émulsion H/E, dont la viscosité serait intermédiaire entre les lotions capillaires trop fluides et d'un usage trop limité, et les crèmes H/E trop visqueuses et présentant un coté gras et collant, tout en conservant les propriétés pro-pénétrantes du glycol.

La présente invention concerne donc une nouvelle composition sous forme d'émulsion fluide de type huile dans eau (H/E) pour une application topique, comprenant entre 30 à 50 % en poids par rapport au poids total de la composition d'au moins un glycol, un système émulsionnant approprié et au moins un agent actif.

Par émulsion fluide, on entend d'une manière avantageuse une émulsion dont la viscosité est comprise entre 3 et 10 Pa.s (3000 et 10000 centipoises), viscosité mesurée avec un appareil Brookfield modèle LVDV II + mobile n° 4, à une vitesse de 30 tours/mn pendant 30 secondes et à une température de 25 °C +/- 3 °C.

i D'une manière avantageuse, on obtient une émulsion stable selon l'invention en sélectionnant comme système émulsionnant approprié au moins un émulsionnant polymérique. Les émulsionnants polymériques sont notamment décrits par CLYMANS & BRAND dans "Cosmetics and Toiletries (manufacture worldwide, 1995, 119-125).

Il s'agit en particulier de polymères amphiphiles anioniques, plus particulièrement ceux comprenant au moins un motif hydrophile de type acide carboxylique insaturé oléfinique, et au moins un motif hydrophobe de type ester d'alkyle en C₁₀-C₃₀.

Selon l'invention, on entend désigner par motifs acryliques des motifs de formule 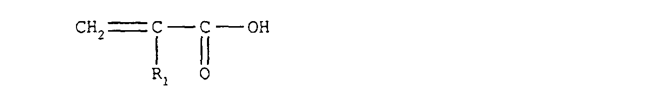 dans laquelle R₁ désigne H ou CH₃ ou C₂H₅, c'est-à-dire des motifs acide acrylique, acide méthacrylique ou acide éthacrylique.

On entend également désigner par motifs acrylates d'alkyles des motifs de structure 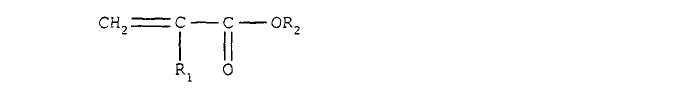 dans laquelle R₁ désigne H ou CH₃ ou C₂H₅, c'est-à-dire des motifs acrylates, méthacrylates ou éthacrylates, R₂ désignant un radical alkyle en C₁₀-C₃₀, de préférence en C₁₂-C₂₂.

Des acrylates conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

D'une manière préférentielle, les polymères amphiphiles anioniques ci-dessus sont réticulés avec un monomère polymérisable réticulant, contenant un groupe CH₂=C< avec au moins un autre groupement polymérisable dont les liaisons insaturées sont non conjuguées l'une par rapport à l'autre.

Des monomères polymérisables réticulants du type sont par exemple, et de préférence, des polyallyléthers tels que notamment le polyallylsucrose et le polyallylpentaérythritol.

Les polymères réticulés de ce type sont bien connus; ils sont notamment décrits dans les brevets US-3 915 921 et US 4 509 949.

Selon l'invention, on peut plus particulièrement utiliser comme polymères amphiphiles anioniques, (i) ceux qui sont constitués de 95 à 60% en poids de motifs acryliques, de 4 à 40% en poids de motifs acrylates et de 0,1 à 6% en poids de monomère réticulant, ou (ii) ceux qui sont constitués de 98 à 96% en poids de motifs acryliques, de 1 à 4% en poids de motifs acrylates et de 0,1 à 0,6% en poids de monomère réticulant.

Parmi lesdits polymères réticulés ci-dessus, on préfère tout particulièrement selon la présente invention, les produits vendus par la société GOODRICH sous les dénominations commerciales PEMULEN TR1, PEMULEN TR2, CARBOPOL 1342, ou CARBOPOL 1382.

La composition selon l'invention comprend avantageusement jusqu'à 1% en poids de système émulsionnant approprié, de préférence entre 0,2 et 0,4 % en poids, par rapport au poids total de la composition.

Le glycol pro-pénétrant est avantageusement choisi parmi le propylène glycol, le dipropylène glycol, le propylène glycol dipelargonate, le lauroglycol ou l'éthoxydiglycol.

De manière préférentielle, la composition selon l'invention comprend entre 40 et 50 % en poids de glycol pro-pénétrant.

Parmi les agents actifs, on peut citer à titre d'exemple les agents modulant la différenciation et/ou la prolifération et/ou la pigmentation cutanée tels que l'acide rétinoïque et ses isomères, le rétinol et ses esters, le rétinal, les rétinoïdes, notamment ceux décrits dans les demandes de brevet FR 2 570 377, EP 199 636, EP 325 540, EP 402 072, la vitamine D et ses dérivés, les estrogènes tels que l'estradiol, l'acide kojique ou l'hydroquinone; les antibactériens tels que le phosphate de clindamycine, l'érythromycine ou les antibiotiques de la classe des tétracyclines; les antiparasitaires, en particulier le métronidazole, le crotamiton ou les pyréthrinoïdes; les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox; les agents anti-inflammatoires stéroïdiens, tels que l'hydrocortisone, les anthralines (dioxyanthranol), les anthranoïdes, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique; les agents anesthésiques tels que le chlorhydrate de lidocaïne et ses dérivés; les agents antiprurigineux comme la thénaldine, la triméprazine ou la cyproheptadine; les agents antiviraux tels que l'acyclovir; les agents kératolytiques tels que les acides alpha- et bêta-hydroxy-carboxyliques ou bêta-cétocarboxyliques, leurs sels, amides ou esters et plus particulièrement les hydroxyacides tels que l'acide glycolique, l'acide lactique, l'acide malique, l'acide salicylique, l'acide citrique et de manière générale les acides de fruits, et l'acide n-octanoyl-5-salicylique; les agents anti-radicaux libres, tels que l'alpha-tocophérol ou ses esters, les superoxyde dismutases, certains chélatants de métaux ou l'acide ascorbique et ses esters; les antiséborrhéiques tels que la progestérone; les antipelliculaires comme l'octopirox ou la pyrithione de zinc; les antiacnéiques comme l'acide rétinoïque, le peroxyde de benzoyle ou l'adapalène; les antimétabolites; les agents pour lutter contre la chute des cheveux comme le monoxidil; les antiseptiques.

Avantageusement, la composition selon l'invention comprend entre 0,0001 et 20 % en poids par rapport au poids total de la composition d'un agent actif, de préférence entre 0,025 et 15 % en poids.

Bien entendu, la quantité d'actif dans la composition selon l'invention dépendra de l'actif considéré. Ainsi, pour un anti-inflammatoire stéroïdien, la composition selon l'invention comprendra avantageusement moins de 1 % en poids d'agent actif, de préférence entre 0,025 et 0,05 % en poids. Pour les hydroquinones, la composition selon l'invention comprendra de préférence entre 2 et 5 % d'agent actif. Pour les antibactériens ou antifongiques comme l'éconazole, la composition selon l'invention comprendra de préférence entre 8 et 10 % en poids d'agent actif.

La phase grasse de l'émulsion selon l'invention peut comprendre des corps gras usuellement utilisés dans le domaine d'application envisagé.

Parmi ceux-ci, on peut citer les corps gras siliconés tels que les huiles de silicone, ainsi que les corps gras non siliconés tels que les huiles végétales, minérales, animales ou synthétiques.

Parmi les corps gras siliconés, on peut citer :
- (i) les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA),
- (ii) les huiles siliconées volatiles, telles que
   - les silicones volatiles cycliques ayant de 3 à 8 atomes de silicium et de préférence de 4 à 5. Il s'agit par exemple de la cyclotétradiméthylsiloxane, de la cyclopentadiméthylsiloxane ou de la cyclohexadiméthylsiloxane,
   - les cyclocopolymères du type diméthylsiloxane/méthylalkylsiloxane, tels que la SILICONE FZ 3109 vendue par la société UNION CARBIDE , qui est un cyclocopolymère diméthylsiloxane/méthyloctylsiloxane,
   - les silicones volatiles linéaires ayant de 2 à 9 atomes de silicium. Il s'agit par exemple de l'hexaméthyldisiloxane, de l'hexyl heptaméthyltrisiloxane ou de l'octyl heptaméthyltrisiloxane,
- (iii) les huiles de silicone phénylées, notamment celles de formule : 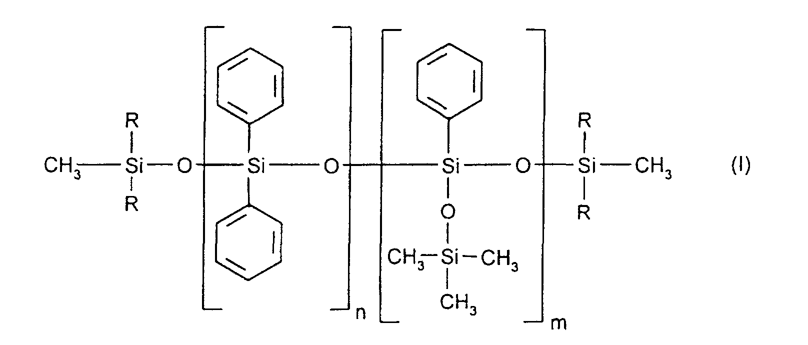
dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme est comprise entre 1 et 100.

Parmi les corps gras non siliconés, on peut citer les huiles usuelles, telles que l'huile de paraffine, de vaseline, l'huile d'almond, le perhydrosqualène, l'huile d'abricot, l'huile de germes de blé, d'amande douce, de calophyllum, de palme, de ricin, d'avocat, de jojoba, d'olive ou de germes de céréales; des esters d'acides gras ou d'alcools gras, telles que l'octyl dodécyl myristate ou les benzoates d'alkyle en C₁₂-C₁₅, des alcools; des acétylglycérides; des octanoates, décanoates ou ricinoléates d'alcools ou de polyalcools; des triglycérides d'acides gras; des glycérides; le polyisobutène hydrogéné, des huiles hydrogénées concrètes à 25°C; des lanolines; des esters gras concrets à 25°C.

Ces corps gras peuvent en particulier être choisis de manière variée par l'homme du métier afin de préparer une composition ayant les propriétés souhaitées, par exemple en consistance ou en texture.

Ainsi, la phase grasse de l'émulsion selon l'invention peut être présente à une teneur comprise entre 5 et 50 % en poids par rapport au poids total de la composition et de préférence comprise entre 15 et 25 % en poids.

La phase aqueuse de l'émulsion selon l'invention peut comprendre de l'eau, une eau florale telle que l'eau de bleuet, ou une eau thermale ou minérale naturelle, par exemple choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avène ou l'eau d'Aix les Bains.

Ladite phase aqueuse peut être présente à une teneur comprise entre 10 et 70 % en poids par rapport au poids total de la composition, de préférence comprise entre 20 et 40 % en poids.

Le pH de la composition selon l'invention est avantageusement compris entre 5 et 7, de préférence compris entre 5,5 et 6,5. Il sera ajusté à la valeur désirée par l'addition de bases ou d'acides usuels, minéraux ou organiques.

Par ailleurs, la composition selon l'invention peut comprendre entre 0 et 3 % en poids, de préférence entre 0 et 2 % en poids, par rapport au poids total de la composition, d'au moins un co-émulsionnant qui peut être choisi parmi les esters d'acides gras saturés ou insaturés, naturels ou synthétiques, notamment de l'acide oléique ou de l'acide (iso)stéarique, tels que les esters de polyglycérine et d'acide isostéarique commercialisés sous la marque LAMEFORM TGI par la société SIDOBRE-SINNOVA HENKEL, l'isostéarate de sorbitan commercialisé sous la marque ARLACEL 987 par la société ICI, le sesquioléate de sorbitan commercialisé sous la marque ARLACEL 83 par la société ICI, les esters de glycol et de l'acide isostéarique comme l'isostéarate de PEG-6 commercialisé sous la marque OLEPAL ISOSTEARIQUE par la société GATTEFOSSE, les esters de sorbitol et d'acide oléique, comme les polysorbates commercialisés sous la marque TWEEN par la société ICI, les éthers d'alcool gras, notamment de l'alcool oléique, en particulier les esters de glycol et d'alcool oléique, comme les oleths commercialisés sous la marque BRIJ par la société ICI, le monostéarate de sorbitan oxyéthyléné, les alcools gras tels que l'alcool stéarylique ou l'alcool cétylique.

En outre, la composition selon l'invention peut comprendre au moins un agent gélifiant et/ou épaississant dans des concentrations préférentielles comprises entre 0 et 5 % en poids, par rapport au poids total de la composition.

L'agent gélifiant et/ou épaississant peut être choisi parmi
- les biopolymères polysaccharidiques comme la gomme de xanthane, la gomme de caroube, la gomme de guar, la gomme de xanthane, les alginates, les celluloses modifiées telles que l'hydroxyéthylcellulose, la méthylcellulose, l'hydroxypropylcellulose, l'hydroxypropylméthylcellulose et la carboxyméthylcellulose,
- les polymères synthétiques comme les acides polyacryliques tels que les polymères poly(méth)acrylates de glycéryl tels que l'HISPAGEL ou le LUBRAGEL des sociétés HISPANO QUIMICA ou GARDIAN, la polyvinylpyrrolidone, l'alcool polyvinylique, les polymères réticulés d'acrylamide et d'acrylate d'ammonium tels que le PAS 5161 ou BOZEPOL C de HOECHST, les polymères réticulés d'acrylamide et d'acide 2-acrylamido 2-méthylpropane sulfonique partiellement ou totalement neutralisé tels que le SEPIGEL 305 de SEPPIC, les polymères réticulés d'acrylamide et de chlorure de méthacryloyloxyéthyltriméthylammonium tels que le SALCARE SC 92 de ALLIED COLLOIDS, les polymères réticulés d'acide acrylique et d'alkyl éthers de succrose ou de pentaérythritol (carbomères) tels que les CARBOPOL 910 à 934 de GOODRICH.

L'émulsion peut comprendre en outre tout additif usuellement utilisé dans le domaine cosmétique ou pharmaceutique, tel que des antioxydants, des colorants, des parfums, des huiles essentielles, des conservateurs, des actifs cosmétiques, des hydratants, des vitamines, des acides gras essentiels, des sphingolipides, des composés auto-bronzants tels que la DHA, des filtres solaires, des polymères liposolubles notamment hydrocarbonés, tels que le polybutène, les polyalkylènes, les polyacrylates et les polymères siliconés compatibles avec les corps gras. Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Ces additifs peuvent être présents dans la composition à raison de 0 à 10% en poids par rapport au poids total de la composition.

Les exemples de formulations ci-dessous permettent d'illustrer les compositions selon l'invention, sans toutefois en limiter la portée. Les quantités des constituants sont exprimées en % en poids par rapport au poids total de la composition.

### Exemple 1 Exemple de formulation selon l'invention

| COMPOSITION | % en poids |
|---|---|
| Eau purifiée | qs 100 |
| Hydroxypropylméthylcellulose | 0,10 |
| Propylène glycol | 47,50 |
| Agent actif | 0,05 |
| Paraffine liquide 110-230 | 20,00 |
| Acrylate / C10-C30 alkyl acrylate crosspolymère (commercialisé sous la marque PEMULEN TR-2 par la société GOODRICH) | 0,30 |
| PEG-6 isotéarate | 2,00 |
| NaOH à 10 % | qs pH 6 |

Dans cette formulation, l'agent actif est resté stable pendant au moins 3 mois à 40 °C.

### Exemple 2 Activité de la formule au propionate de clobétasol

La formule selon l'invention ci-dessus a été reprise avec du propionate de clobétasol comme agent actif.

Des tests de vasoconstriction selon un protocole de Stoughton modifié ont été effectués en comparaison avec la crème H/E correspondante commercialisée sous la marque TEMOVATE par la société GLAXO.

Les résultats montrent une activité identique pour les deux formules, venant confirmer que malgré la modification de la viscosité de la formulation selon l'invention et l'emploi d'un système émulsionnant différent, le glycol pro-pénétrant a conservé ses propriétés pro-pénétrantes.

## Revendications

1. Composition sous forme d'émulsion fluide de type huile dans eau (H/E) pour une application topique, comprenant entre 30 à 50 % en poids par rapport au poids total de la composition d'au moins un glycol pro-pénétrant et au moins un agent actif, **caractérisée en ce qu'**elle comprend un système émulsionnant approprié, qui comprend au moins un polymère amphiphile anionique comprenant au moins un motif hydrophile de type acide carboxylique insaturé oléfinique, et au moins un motif hydrophobe de type ester d'alkyle en C₁₀-C₃₀, ladite composition ayant une viscosité comprise entre 3 et 10 Pa.s (3000 et 10000 centipoises), viscosité mesurée avec un appareil Brookfield modèle LVDV II + mobile n° 4, à une vitesse de 30 tours/mn pendant 30 secondes et à une température de 25 °C +/- 3 °C.

2. Composition selon la revendication 1, **caractérisée en ce que** les polymères amphiphiles anioniques sont réticulés avec un monomère polymérisable réticulant, contenant un groupe CH₂=C< avec au moins un autre groupement polymérisable dont les liaisons insaturées sont non conjuguées l'une par rapport à l'autre en particulier avec des polyallyléthers tels que le polyallylsucrose et le polyallylpentaérythritol. '

3. Composition selon l'une des revendications 1 ou 2, **caractérisée en ce que** le polymère amphiphile anionique est choisi parmi (i) ceux qui sont constitués de 95 à 60% en poids de motifs acryliques, de 4 à 40% en poids de motifs acrylates et de 0,1 à 6% en poids de monomère réticulant, ou (ii) ceux qui sont constitués de 98 à 96% en poids de motifs acryliques, de 1 à 4% en poids de motifs acrylates et de 0,1 à 0,6% en poids de monomère réticulant.

4. Composition selon l'une des revendications 1 à 3, **caractérisée en ce qu'**elle comprend jusqu'à 1 % en poids de système émulsionnant approprié, de préférence entre 0,2 et 0,4 % en poids.

5. Composition selon l'une des revendications 1 à 4, **caractérisée en ce que** le glycol pro-pénétrant est choisi parmi le propylène glycol, le dipropylène glycol, le propylène glycol dipelargonate, le lauroglycol ou l'éthoxydiglycol.

6. Composition selon l'une des revendications 1 à 5, **caractérisée en ce qu'**elle comprend entre 40 et 50 % en poids de glycol pro-pénétrant.

7. Composition selon l'une des revendications 1 à 6, **caractérisée en ce que** l'agent actif est choisi parmi les agents modulant la différenciation et/ou la prolifération et/ou la pigmentation cutanée, les antibactériens, les antiparasitaires, les antifongiques, les agents anti-inflammatoires stéroïdiens ou les agents anti-inflammatoires non-stéroïdiens, les agents anesthésiques, les agents antiprurigineux, les agents antiviraux, les agents kératolytiques, les agents anti-radicaux libres, les antiséborrhéiques, les antipelliculaires, les antiacnéiques, les antimétabolites, les agents pour lutter contre la chute des cheveux, et les antiseptiques.

8. Composition selon l'une des revendications 1 à 7, **caractérisée en ce qu'**elle comprend entre 0,0001 et 20 % en poids par rapport au poids total de la composition d'au moins un agent actif, de préférence entre 0,025 et 15 % en poids.

9. Composition selon l'une des revendications 1 à 8, **caractérisée en ce que** la phase grasse est présente à une teneur comprise entre 5 et 50 % en poids par rapport au poids total de la composition, de préférence comprise entre 15 et 25 % en poids.

10. Composition selon l'une des revendications 1 à 9, **caractérisée en ce que** la phase aqueuse est présente à une teneur comprise entre 10 et 70 % en poids par rapport au poids total de la composition, de préférence comprise entre 20 et 40 % en poids.

11. Composition selon l'une des revendications 1 à 10, **caractérisée en ce qu'**elle comprend entre 0 et 3 % en poids par rapport au poids total de la composition, de préférence entre 0 et 2 % en poids d'au moins un co-émulsionnant.

12. Composition selon l'une des revendications 1 à 11, **caractérisée en ce qu'**elle comprend, au moins un agent gélifiant et/ou épaississant dans des concentrations comprises entre 0 et 5 % en poids, par rapport au poids total de la composition.

## Patentansprüche

1. Zusammensetzung in Form einer fluiden Emulsion vom Typ Öl in Wasser (O/W) für eine topische Anwendung, die 30 bis 50 Gew.-% mindestens eines penetrationsfördernden Glykols, bezogen auf das Gesamtgewicht der Zusammensetzung, und mindestens einen Wirkstoff enthält, **dadurch gekennzeichnet, dass** sie ein geeignetes emulgierendes System aufweist, das mindestens ein anionisches amphiphiles Polymer enthält, welches mindestens eine hydrophile Einheit vom Typ einer olefinisch ungesättigten Carbonsäure und mindestens eine hydrophobe Einheit vom Typ eines C₁₀₋₃₀-Alkylesters aufweist, wobei die Zusammensetzung eine mit einem Brookfield-Gerät, Modell LVDV II mit mobilem Teil Nr. 4, bei einer Geschwindigkeit von 30 U/min und einer Temperatur von 25 °C ± 3 °C während einer Zeitspanne von 30 Sekunden bestimmte Viskosität im Bereich von 3 bis 10 Pa·s (3 000 bis 10 000 Centipoise) aufweist.

2. Zusammensetzung nach Anspruch 1, **dadurch gekennzeichnet, dass** die anionischen amphiphilen Polymere mit einem vernetzenden polymerisierbaren Monomer, das eine Gruppe CH₂=C< und mindestens eine weitere polymerisierbare Gruppe enthält, deren ungesättigte Bindungen nicht konjugiert sind, und insbesondere mit Polyallylethern, wie Polyallylsaccharose und Polyallylpentaerythrit, vernetzt sind.

3. Zusammensetzung nach einem der Ansprüche 1 oder 2, **dadurch gekennzeichnet, dass** das anionische amphiphile Polyrner unter (i) den anionischen amphiphilen Polymeren, die zu 95 bis 60 Gew.-% aus Acryleinheiten, zu 4 bis 40 Gew.-% aus Acrylateinheiten und zu 0,1 bis 6 Gew.-% aus vernetzendem Monomer bestehen, oder (ii) den anionischen amphiphilen Polymeren, die zu 98 bis 96 Gew.-% aus Acryleinheiten, zu 1 bis 4 Gew.-% aus Acrylateinheiten und zu 0,1 bis 0,6 Gew.-% aus vernetzendem Monomer bestehen, ausgewählt ist.

4. Zusammensetzung nach einem der Ansprüche 1 bis 3, **dadurch gekennzeichnet, dass** sie bis zu 1 Gew.-% und vorzugsweise 0,2 bis 0,4 Gew.-% eines geeigneten emulgierenden Systems enthält.

5. Zusammensetzung nach einem der Ansprüche 1 bis 4, **dadurch gekennzeichnet, dass** das penetrationsfördernde Glykol unter Propylenglykol, Dipropylenglykol, Propylenglykoldipelargonat, Lauroglycol oder Ethoxydiglykol ausgewählt ist.

6. Zusammensetzung nach einem der Ansprüche 1 bis 5, **dadurch gekennzeichnet, dass** sie 40 bis 50 Gew.-% eines penetrationsfördernden Glykols enthält.

7. Zusammensetzung nach einem der Ansprüche 1 bis 6, **dadurch gekennzeichnet, dass** der Wirkstoff ausgewählt ist unter Wirkstoffen, die die Differenzierung und/oder Proliferation und/oder Pigmentierung der Haut modulieren, antibakteriellen Wirkstoffen, antiparasitären Wirkstoffen, Antimykotika, steroidalen entzündungshemmenden Wirkstoffen oder nichtsteroidalen entzündungshemmenden Wirkstoffen, Anästhetika, juckreizstillenden Wirkstoffen, antiviralen Wirkstoffen, Keratolytika, Radikalfängern für freie Radikale, Antiseborrhoeika, Antischuppenmitteln, Wirkstoffen gegen Akne, Antimetaboliten, Wirkstoffen zur Bekämpfung von Haarausfall, sowie Antiseptika.

8. Zusammensetzung nach einem der Ansprüche 1 bis 7, **dadurch gekennzeichnet, dass** sie bezogen auf das Gesamtgewicht der Zusammensetzung 0,0001 bis 20 Gew.-% und vorzugsweise 0,025 bis 15 Gew.-% mindestens eines Wirkstoffs enthält.

9. Zusammensetzung nach einem der Ansprüche 1 bis 8, **dadurch gekennzeichnet, dass** die Fettphase in einem Mengenanteil von 5 bis 50 Gew.-% und vorzugsweise von 15 bis 25 Gew.-%, bezogen auf das Gesamtgewicht der Zusammensetzung, vorliegt.

10. Zusammensetzung nach einem der Ansprüche 1 bis 9, **dadurch gekennzeichnet, dass** die wässerige Phase in einem Mengenanteil von 10 bis 70 Gew.-% und vorzugsweise von 20 bis 40 Gew.-%, bezogen auf das Gesamtgewicht der Zusammensetzung, vorliegt.

11. Zusammensetzung nach einem der Ansprüche 1 bis 10, **dadurch gekennzeichnet, dass** sie bezogen auf das Gesamtgewicht der Zusammensetzung 0 bis 3 Gew.-% und vorzugsweise 0 bis 2 Gew.-% mindestens eines Coemulgators enthält.

12. Zusammensetzung nach einem der Ansprüche 1 bis 11, **dadurch gekennzeichnet, dass** sie mindestens einen Gelbildner und/oder ein Verdickungsmittel in Mengenanteilen enthält, die im Bereich von 0 bis 5 Gew.-%, bezogen auf das Gesamtgewicht der Zusammensetzung, liegen.

## Claims

1. Composition in the form of an oil-in-water (0/W) type fluid emulsion for topical application, comprising between 30 and 50% by weight relative to the total weight of the composition of at least one pro-penetrating glycol and at least one active agent, **characterized in that** it comprises an appropriate emulsifying system which comprises at least one anionic amphiphilic polymer comprising at least one hydrophilic unit of the olefin unsaturated carboxylic acid type, and at least one hydrophobic unit of the C₁₀-C₃₀ alkyl ester type, the said composition having a viscosity of between 3 and 10 Pa.s (3000 and 10,000 centipoises), a viscosity measured with a Brookfield apparatus model LVDV II + rotor No. 4, at a speed of 30 revolutions/min for 30 seconds and at a temperature of 25°C ± 3°C.

2. Composition according to Claim 1, **characterized in that** the anionic amphiphilic polymers are crosslinked with a cross-linking polymerizable monomer containing a CH₂=C< group with at least one other polymerizable group whose unsaturated bonds are not conjugated relative to each other in particular with polyallyl ethers such as polyallylsucrose and polyallylpentaerythritol.

3. Composition according to either of Claims 1 and 2, **characterized in that** the anionic amphiphilic polymer is chosen from (i) those which consist of 95 to 60% by weight of acrylic units, of 4 to 40% by weight of acrylate units and of 0.1 to 6% by weight of cross-linking monomer, or (ii) those which consist of 98 to 96% by weight of acrylic units, of 1 to 4% by weight of acrylate units and of 0.1 to 0.6% by weight of cross-linking monomer.

4. Composition according to one of Claims 1 to 3, **characterized in that** it comprises up to 1% by weight of appropriate emulsifying system, preferably between 0.2 and 0.4% by weight.

5. Composition according to one of Claims 1 to 4, **characterized in that** the pro-penetrating glycol is chosen from propylene glycol, dipropylene glycol, propylene glycol dipelargonate, lauroglycol or ethoxydiglycol.

6. Composition according to one of Claims 1 to 5, **characterized in that** it comprises between 40 and 50% by weight of pro-penetrating glycol.

7. Composition according to one of Claims 1 to 6, **characterized in that** the active agent is chosen from the agents modulating skin differentiation and/or proliferation and/or pigmentation, antibacterial agents, antiparasitic agents, antifungal agents, steroidal anti-inflammatory agents or non-steroidal anti-inflammatory agents, anaesthetic agents, antipruritic agents, antiviral agents, keratolytic agents, anti-free radical agents, antiseborrhoeic agents, antidandruff agents, anti-acne agents, antimetabolites, agents for combating hair loss, and antiseptics.

8. Composition according to one of Claims 1 to 7, **characterized in that** it comprises between 0.0001 and 20% by weight relative to the total weight of the composition of at least one active agent, preferably between 0.025 and 15% by weight.

9. Composition according to one of Claims 1 to 8, **characterized in that** the fatty phase is present in an amount of between 5 and 50% by weight relative to the total weight of the composition, preferably between 15 and 25% by weight.

10. Composition according to one of Claims 1 to 9, **characterized in that** the aqueous phase is present in an amount of between 10 and 70% by weight relative to the total weight of the composition, preferably between 20 and 40% by weight.

11. Composition according to one of Claims 1 to 10, **characterized in that** it comprises between 0 and 3% by weight relative to the total weight of the composition, preferably between 0 and 2% by weight, of at least one co-emulsifier.

12. Composition according to one of Claims 1 to 11, **characterized in that** it comprises at least one gelling and/or thickening agent in concentrations of between 0 and 5% by weight relative to the total weight of the composition.
